(19) Europäisches Patentamt
European Patent Office
Office européen des brevets

(11) Veröffentlichungsnummer: **0 078 544**
**A1**

(12) # EUROPÄISCHE PATENTANMELDUNG

(21) Anmeldenummer: **82110130.0**

(22) Anmeldetag: **03.11.82**

(51) Int. Cl.³: **G 01 N 33/28**, G 01 N 31/22

(30) Priorität: **03.11.81 DE 3143589**
**26.02.82 DE 3207027**

(43) Veröffentlichungstag der Anmeldung: **11.05.83**
**Patentblatt 83/19**

(84) Benannte Vertragsstaaten: **AT CH DE FR GB IT LI SE**

(71) Anmelder: **LUCAS INDUSTRIES public limited company, Great King Street, Birmingham, B19 2XF (GB)**

(72) Erfinder: **Förster, Joachim, Cheruskerstrasse 121, D-4800 Bielefeld 14 (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

(54) **Mittel und Verfahren zur Anzeige eines kritischen Wassergehalts in Hydraulikflüssigkeiten, insbesondere Bremsflüssigkeiten.**

(57) Die Erfindung betrifft ein Mittel und dessen Anwendungsverfahren zur Anzeige eines kritischen Wassergehalts in einer Hydraulikflüssigkeit, insbesondere einer Bremsflüssigkeit – der im allgemeinen in der Grössenordnung von 2% liegt –, mit Hilfe einer Indikatorlösung enthaltend Diphenylcarbazon und 1,10-Phenanthrolin bzw. dessen Additionssalz oder mit Hilfe von Monoazo-, Disazo- oder Trisazo-Farbstoffen, die durch Übergang von der dissoziierten Form in die dissoziierte eine Farbänderung erfahren.

EP 0 078 544 A1

ACTORUM AG

0078544

PATENTANWÄLTE
WUESTHOFF - v. PECHMANN - BEHRENS - GOETZ
EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

D-8000 MÜNCHEN 90
SCHWEIGERSTRASSE 2
TELEFON: (089) 66 20 51
TELEGRAMM: PROTECTPATENT
TELEX: 524 070



EP-56 697

Anm.: Lucas Industries public limited company

## Beschreibung

Farbstoffe bzw. Indikatoren zur Bestimmung des Wassergehaltes von Hydraulikflüssigkeiten, insbesondere Bremsflüssigkeiten

Hydraulische Flüssigkeiten bestehen im allgemeinen aus Mineralölen oder synthetischen Produkten auf der Basis von Polyglykolen und/oder Polyglykolethern der allgemeinen Formel

$$R_x-(OC_2H_4)_nX,$$

worin X die Gruppe $-OCH_3$, $OC_2H_5$ oder $OC(CH_3)_3$ sein kann; also Alkyl-polyethylenglykol-tert.butylethern, in denen die Alkylgruppe geradkettig oder verzweigtkettig sein kann und 1 bis 4 C-Atome enthält. Die Anzahl der Ethylenglykol-Einheiten n liegt im allgemeinen zwischen 2 und 10 (siehe hierzu beispielsweise die in der DE-AS 17 68 933 angegebenen Stoffe).

Es ist bekannt, daß diese Produkte dazu neigen, Wasser aufzunehmen, d. h. sie sind hygroskopisch. Der Siedepunkt der wasserfreien Produkte liegt in der Größenordnung von etwa 280 °C. Durch die Hygroskopizität der Grundsubstanzen der hydraulischen Flüssigkeiten nimmt mit zunehmender Betriebszeit deren Wassergehalt ständig zu. Mit steigendem Wassergehalt sinkt aber der Siedepunkt der Hydraulikflüssigkeiten erheblich. Ein Wassergehalt von nur etwa 2 % in einer Hydraulikflüssigkeit führt bereits zu einer Siedepunktserniedrigung von beispielsweise 280 °C auf etwa 200 °C. Der Siedepunkt kann bei einem entsprechenden Wassergehalt bis auf etwa 150 °C und darunter sinken.

Es ist bekannt, daß durch diese Tendenz der Hydraulikflüssigkeiten aufgrund des Wassergehalts die Funktionsfähigkeit der damit ausgestatteten Maschinen und Aggregate, insbesondere die Betriebssicherheit von Kraftfahrzeugbremsen, weitgehend eingeschränkt wird. Es ist ebenfalls bekannt, daß Hydraulikaggregate, vor allem Kraftfahrzeugbremsen, in kurzer Zeit sehr hohe Temperaturen erreichen können. Dabei kommt es zu einer Verdampfung des Wassers in der Hydraulikflüssigkeit; eine sich daraus ergebende Blasenbildung kann zu einem vollständigen Versagen einer Bremse führen.

Aus dem SAE-Papier Nr. 680007 vom 8. Januar 1968 ist es auch bekannt, daß mit zunehmender Luftfeuchtigkeit der Wassergehalt der Bremsflüssigkeiten zunimmt. So liegt bei 25 % relativer Luftfeuchtigkeit der Wassergehalt der Bremsflüssigkeit bei etwa 2 %, was einem Siedepunkt von ungefähr 170 °C entspricht. Bei 50 % relativer Luftfeuchtigkeit steigt der Wassergehalt auf 6 % bis 7 % an und der Siedepunkt fällt auf etwa 125 °C ab. Steigt die relative Luftfeuchtigkeit auf 75 % an, wird die Sättigung von etwa 17 % Wasser in der Bremsflüssigkeit erreicht; der Siedepunkt liegt dann nur noch knapp über dem des Wassers, nämlich bei ca. 108 °C. Es ist zu erwarten, daß im Hochgebirge - niedriger Druck - die Siedetemperatur auch unter 100 °C abfallen kann.

/3

Wenn auch die Bremsflüssigkeit in den Kraftfahrzeugen überwiegend nicht der Umgebungsluft direkt ausgesetzt ist, so ist sie in den Zylindern etc. für die Feuchtigkeit infolge Diffusion durch Gummiteile doch allmählich zugänglich. Auch sind die Bremsflüssigkeiten im Vorratsbehälter bei vielen Konstruktionen durch die kleine Bohrung für den Druckausgleich mit der Umgebungsluft direkt verbunden. Die Wasseraufnahme bedarf zwar einer gewissen Zeit, doch haben Untersuchungen gezeigt, daß bereits nach etwa 100 Tagen die Bremsflüssigkeiten mit Wasser in Abhängigkeit von der Luftfeuchtigkeit gesättigt sind. Je nach Zusammensetzung der Flüssigkeit und bei geringerer relativer Luftfeuchtigkeit kann hierbei der Siedepunkt noch bei 118 °C liegen. Bei längerer höherer Luftfeuchtigkeit kann er aber auch auf knapp über 100 °C abgefallen sein, ein Temperaturwert der sehr leicht in der Bremsanlage beim Bremsen erreicht oder überschritten werden kann.

Es ist auch bekannt, den Wassergehalt von Flüssigkeiten durch die Bestimmung der elektrischen Leitfähigkeit - oder deren Kehrwert, des spezifischen Widerstands - zu ermitteln. Ein dafür vorgesehenes Meßgerät ist aus der DE-OS 29 29 784 bekannt.

Es ist auch bekannt, durch Verfolgung des Wertes der elektrischen Leitfähigkeit einer Hydraulikflüssigkeit das Erreichen des kritischen Wassergehaltes festzustellen.

Die Leitfähigkeitswerte einer Hydraulikflüssigkeit mit 0 bis etwa 2 % Wasser sind sehr gering. Nach der DE-OS 31 34 954 wird der elektrische Widerstand einer Hydraulikflüssigkeit, insbesondere Bremsflüssigkeit, - der in der Größenordnung von 10 $k\Omega/cm^3$ liegt - wesentlich herabgesetzt und damit die Leitfähigkeit erhöht durch einen Zusatz an in der Flüssigkeit und eventuell vorhandenem

Wasser unter Ionenbildung löslichen aliphatischen Mono- oder Polycarbonsäuren und Oxycarbonsäuren - wie Milchsäure und Citronensäure - oder aromatischen Mono- oder Dicarbonsäuren und Hydroxycarbonsäuren und deren Salze, (Thio)Acetamid oder Ethanolaminen, wobei sich ein Salz der Salicylsäure als besonders geeignet erwiesen hat.

Aufgabe der Erfindung ist ein verbesserter Indikator bzw. Farbstoff aus der Klasse der Azo-Farbstoffe zur Feststellung, wann eine hydraulische Flüssigkeit, insbesondere eine Bremsflüssigkeit, einen kritischen Wassergehalt erreicht.

Diese Aufgabe wird entweder a) mit Hilfe eines Indikators in Form eines Gemischs von Diphenylcarbazon und 1,10-Phenanthrolin oder deren Additionssalz oder b) mit Hilfe eines Monoazo-, Disazo- oder Trisazo-Farbstoffs der allgemeinen Formel

$$(MO_3S)_x-Ar-N=N-Ar_1(SO_3M)_y-Z$$

gelöst, worin Ar, $Ar_1$ und $Ar_2$ eine Phenylen- oder Naphthylengruppe, gegebenenfalls substituiert mit einer oder mehreren Hydroxyl- und/oder Aminogruppe(n), Z ein Wasserstoffatom oder die Gruppe $-N=N-Ar_2(SO_3M)_z$ ist und M Na, K oder $NH_4$ sein kann und x, y und z 0 bis 3 ist.

Der Indikator bei der Variante a) kann in einem damit imprägnierten Papierstreifen zur Anwendung gelangen. Dieser Indikator wird in wasserfreier Hydraulikflüssigkeit rot und bleicht bei einem Wassergehalt von ~2 % auf hellrosa aus. Rot wird üblicherweise als Anzeige einer Gefahr empfunden - bei diesem Indikator bedeutet jedoch rot gerade den gefahrlosen Zustand und die viel geringere Farbintensität des Hellrosa den Gefahrenzustand.

/5

Der Indikator kann hierbei sowohl in Form einer Lösung in einer Flüssigkeit als auch als Papierstreifen verwendet werden. Erfindungsgemäß besteht der Indikator aus einer Mischung von a) Diphenylcarbazon und b) 1,10-Phenanthrolin oder dessen Additionssalze mit einer anorganischen oder organischen Säure. Es ist zwar aus der US-PS 3 222 292 bekannt, 1-Hydroxy-4-acetamino-antrachinon als "Wasser-Indikator" für Bremsflüssigkeiten zu verwenden, jedoch findet dabei ein Farbumschlag von orangerot in blaurot statt. Dieser Farbumschlag ist in gelblichen Bremsflüssigkeiten nicht oder nicht mit der erforderlichen Sicherheit festzustellen.

Im Falle der Verwendung einer Indikatorflüssigkeit zur Bestimmung des Wassergehalts der Bremsflüssigkeit wird der Indikator in Form einer Lösung in einem flüssigen Kohlenwasserstoff angewendet. Diese rot-violett gefärbte Indikatorlösung wird in einem Behälter, z. B. einem durchsichtigen Glasfläschchen oder Reagenzglas, mit z. B. der gleichen Menge der zu untersuchenden Bremsflüssigkeit in Berührung gebracht, indem die Bremsflüssigkeit mit der Indikatorflüssigkeit überschichtet wird. An der Berührungsfläche zwischen der unteren Phase der Bremsflüssigkeit und der oberen Phase der Farbindikatorflüssigkeit tritt dann bei einer Konzentration von 2 % oder mehr Wasser in der Bremsflüssigkeit im Berührungsbereich eine Entfärbung der rot-violetten Indikatorlösung ein.

Die beiden Flüssigkeiten sollen nach dem Überschichten der Bremsflüssigkeit mit der Indikatorflüssigkeit nicht geschüttelt werden, da sonst der Farbindikator die gesamte Bremsflüssigkeit färben kann und dann die Farbaufhellung durch den Wassergehalt nicht deutlich sichtbar wird. Die Farbaufhellung der Indikatorflüssigkeit an der Phasenberührungsfläche tritt relativ schnell ein, so daß maximal nach 1 Minute festgestellt werden kann, ob in der Bremsflüssigkeit soviel Wasser enthalten ist, daß sie ausgetauscht werden muß.

/6

Zur Herstellung der Farbindikatorflüssigkeit empfiehlt es sich, zunächst eine konzentrierte Indikatorlösung aus den beiden Komponenten und Aceton herzustellen. Hierbei soll die Konzentration des Diphenylcarbazons in dieser Lösung etwa 0,1 - 4,5 Gew.-% und die des Phenanthrolins bzw. des Phenanthrolinsalzes 0,1 - 1 Gew.-% betragen; diese konzentrierte Indikatorlösung wird dann in einer Menge von 0,2 - 0,6 $cm^3$ mit 20 $cm^3$ einer Kohlenwasserstoff-Flüssigkeit, insbesondere Isooctan, n-Hexan oder n-Heptan vermischt. Die so bereitete Farbindikatorflüssigkeit soll 0,003 - 0,045 Gew.-% Diphenylcarbazon und 0,003 - 0,01 Gew.-% Phenanthrolin- bzw. dessen Salz enthalten und wird dann, wie beschrieben, mit der Bremsflüssigkeit in Berührung gebracht, indem man die Indikatorflüssigkeit vorsichtig auf die Bremsflüssigkeit fließen läßt und diese überschichtet.

Im Falle der Anwendung des Farbindikators in Form eines Papierteststreifens wird ein aufsaugfähiges Papier, z. B. Filterpapier, mit der konzentrierten Indikatorlösung imprägniert, worauf man das Aceton verdunsten läßt. Nach dem Trocknen wird das hell-rosa gefärbte Indikatorpapier in Streifen geschnitten. Beim Eintauchen* in die Bremsflüssigkeit nimmt das Indikatorpapier in der wasserfreien Bremsflüssigkeit eine dunkelrote Färbung an. Enthält dagegen die Bremsflüssigkeit 2 % Wasser oder mehr, so bleibt das Indikatorpapier hell-rosa bzw. hellt sich die Färbung des Indikatorpapiers nach kurzer Zeit wieder auf und wird hellrosa. Wenn auch die Verwendung des Indikatorpapierstreifens einfacher ist, so ist allerdings die Verfärbung beim Indikatorpapier nicht so deutlich wie bei Anwendung der Flüssigkeit, bei der die rötlich-violette Indikatorlösung im Bereich der Berührung mit der Bremsflüssigkeit im Falle der Anwesenheit von Wasser sich

* eines solchen Streifens

/7

schnell hellrosa verfärbt bzw. farblos wird. Diese Verfärbung ist deutlicher und schneller zu erkennen als im Falle der Anwendung des Indikators auf dem Papierstreifen.

Der Farbstoff für die Variante b) ist z. B. Fast Yellow SX (13015), Fast red E (16045), Chrysoin SGX, Azorubin S (16185), Naphtholrot S (Amaranth) (16185), Cochenillerot A (16255), Ponceau 6R (16290), Gelb 27175N (13440/445), Orange GGN (15980), Gelborange S und ähnliche wasserlösliche Azofarbstoffe.

Grundsätzlich kann man sagen, daß sich alle Farbstoffe eignen, die eine Färbung entwickeln, wenn der Wassergehalt der hydraulischen Flüssigkeit groß genug wird, um ihre Löslichkeit oder Ionisation zu bewirken. Die anzuwendenden Farbstoffe müssen eine ausreichende Farbtiefe in den hydraulischen Flüssigkeiten, insbesondere Flüssigkeiten bei einem Wassergehalt von 2 % aufweisen, während sie in den wasserfreien Flüssigkeiten praktisch farblos sind.

Voraussetzung für die Anwendbarkeit von Farbstoffen nach der Erfindung ist, daß sie

1. in Wasser löslich sind und in Lösung eine ausreichende Farbtiefe besitzen. Gelbliche Farbtöne sind nicht so sehr erwünscht, da Bremsflüssigkeiten unter Prüfbedingungen möglicherweise gelblich erscheinen. Es ist zu beachten, daß nach den gesetzlichen Vorschriften nichtmineralische Bremsflüssigkeiten farblos bis bräunlich sein dürfen.

2. Bremsflüssigkeiten haben normalerweise einen schwach alkalischen pH-Wert zwischen 7 und 11,5 aufgrund der Anwesenheit von Korrosionsinhibitoren, so daß die Farbstoffe in diesen Bereichen nicht pH-Wert-empfindlich sein sollen.

/8

3. Wie oben angegeben, besitzen die erfindungsgemäß anwendbaren Azofarbstoffe Sulfonatgruppen. Die gleiche Wirksamkeit werden jedoch auch Farbstoffe besitzen, die anstelle der Sulfonatgruppen Halogenidgruppen, insbesondere Chloride, und Carbonsäuresalze tragen oder andere saure oder basische Einheiten innerhalb des Moleküls aufweisen. Der ionische Charakter begünstigt die Wasserlöslichkeit im Gegensatz zur Löslichkeit in organischen Stoffen.

Bevorzugt wird Amaranth, an dem im folgenden die Erfindung weiter erläutert wird. Es ist ein roter Azofarbstoff der Summenformel $C_{20}H_{22}N_2O_{10}S_3Na_3$, der auch unter der Bezeichnung "Naphtholrot S" bekannt ist und der das Trinatriumsalz der 1-(4-Sulfo-1-naphthylazo)2-naphthol-3,6-disulfonsäure ist (C.I. 16185).

Der Farbumschlag dieser Farbstoffe beim Übergang von der ungelösten Form in den gefärbten dissoziierten Zustand erfolgt nicht plötzlich, sondern der Endwert der Farbtiefe wird mit zunehmendem Wassergehalt allmählich erreicht. Entgegen dem obigen Indikator nimmt bei den erfindungsgemäß angewandten Azofarbstoffen die Farbintensität und Farbtiefe mit steigendem Wassergehalt zu und signalisiert damit sehr augenfähig die drohende oder aufziehende Gefahr.

Der Azofarbstoff Amaranth löst sich in organischen Flüssigkeiten nicht oder nur sehr wenig, ist jedoch in Wasser leicht löslich. Er ist daher ein hervorragender Indikator für die Anwesenheit von Wasser. Amaranth oder Naphtholrot S wird mit zunehmendem Wassergehalt der hydraulischen Flüssigkeit zuerst rosé - bei einem Wassergehalt der Flüssigkeit von etwa 1 % - und erfährt mit zunehmendem Wassergehalt eine Erhöhung der Farbintensität oder eine Vertiefung des Farbtons, bis schließlich tiefrot bei etwa 2 % Wasser erreicht ist. Da gerade dies die

kritische Wassermenge für z. B. Bremsflüssigkeiten ist, sind diese Azofarbstoffe für diesen Zweck besonders geeignet.

Infolge der hohen Löslichkeit folgender Farbstoffe in trockenen Bremsflüssigkeiten ist die Intensität der Farbänderung bis 5 % Wassergehalt meist nicht gut, so daß man diese Farbstoffe kaum für derzeit übliche Bremsflüssigkeiten anwenden wird.

| Name | Farbstoffklasse | C.I. |
| --- | --- | --- |
| Janus Green | Monoazo-Verbindungen | 11050 |
| Ponseau de Xylidine (Acid Red 26) | Monoazo-Verbindungen | 16150 |
| Azophloxin (Acid Red II) | Monoazo-Verbindungen | 18050 |
| Congo Red (Direct Red 28) | Disazo-Verbindungen | 22120 |
| Light Green SF (Acid Green 5) | Triarylmethan | 42095 |
| Crystal Violet (Basic Violet 3) | Triarylmethan | 42555 |
| Coriphosphine (Basic Yellow 7) | Acridin | 46020 |
| Azo Carmino G (CX) (Acid Red 101) | Azin | 50085 |
| Celestine (Blue B) (Mordant Blue 14) | Oxazin | 51050 |
| Azure B | Thiazin | 52010 |
| Alizarin Red S (Mordant red 3) | Anthrachinon | 58005 |
| Carmine | Indigoid | 74370 |

Folgende Farbstoffe sind wegen verschiedener Nachteile für die zur Zeit üblichen Bremsflüssigkeiten nicht geeignet.

| Name | Farbstoff-klassen | C.I. |
|---|---|---|
| Solochrome dark blue (mordant Black 17) | Monoazo-Verbindungen (15705) | purpur in Wasser, bräunlich-purpur in Bremsflüssigkeit. die Farben sind nicht ausreichend unterschiedlich |
| Alizarin Yellow GG (mordant yellow 1) | Monoazo-Verbindungen (14025) | keine Farbänderung bei Wasser in der Flüssigkeit |
| Aniline Blue (water soluble) (Acid Blue 22) | Triarylmethan (42755) | nicht ausreichend löslich in wasserfreier und wasserhaltiger Bremsflüssigkeit |
| Methyl Green (Basic Blue 20) | Triarylmethan (42585) | geringe Färbung in Bremsflüssigkeit |
| Berlin Blue (soluble) (Pigment Blue 27) | anorganisch | zu geringe Farbe in wasserfreier und wasserhaltiger Bremsflüssigkeit |

Eine gewisse Farbintensitätsteigerung oder Farbänderung mit in Bremsflüssigkeiten enthaltenem Wasser zeigen folgende Farbstoffe:

| | | |
|---|---|---|
| Pontamine Sky Blue 5BX (Direct Blue 15) | Disazo-Verbindungen (24400) | die Löslichkeit in Bremsflüssigkeit wird durch Wasser verbessert und eine gründe Färbung entwickelt sich; gewisse Ähnlichkeit mit Amaranth, jedoch weniger deutlich |

/11

| Name | Farbstoffklassen | C.I. |
|---|---|---|
| Napthol Green B (Acid Green 1) | Nitroso (10020) | die Löslichkeit steigt mit dem Wassergehalt der Flüssigkeit und führt zu einem tiefgrünen Farbton |
| Methyl Blue (Acid Blue 93) | Triarylmethan (42780) | unlöslich in wasserfreier Flüssigkeit |
| Chrome Azurol 8 (Mordant blue 29) | Triarylmethan (43825) | purpur-violett in wasserfreier Flüssigkeit, schlägt um in orange-braun bei Wasserzusatz |
| Acridine Orange (Basic oragne 14) | Acridin (46005) | steigende Löslichkeit mit Wassergehalt der Flüssigkeit, obwohl eine tiefere Färbung als orange wünschenswert wäre |
| Indigo Carmine (Acid Blue 74) | Indigoid (73015) | Farbumschlag von gelbgrün in wasserfreier Flüssigkeit zu grün in wasserhaltiger Flüssigkeit |

Nach der Erfindung kann der Azofarbstoff als Indikatormittel in einem Prüfstreifen, aus beispielsweise Papier, angewandt werden. Zu dessen Herstellung wird das Farbstoffpulver in einen Flüssigkeit aufsaugenden Papierstreifen eingebracht. Dazu kann man den festen Farbstoff entweder in den Papierstreifen einreiben oder in einer wasserfreien organischen Flüssigkeit - für Amaranth eignet sich dafür Amylalkohol - dispergieren, den Papierstreifen mit der Dispersion tränken und das Dispersionsmittel abdampfen. Man kann aber auch das Farbstoffpulver in einen von zwei Papierstreifen gebildeten Hohlraum einbringen.

/12

Der mit Indikator versehene Prüfstreifen wird in die zu untersuchende hydraulische Flüssigkeit getaucht und dann eine Änderung der Farbintensität proportional dem Wassergehalt der Flüssigkeit festgestellt. Das erfindungsgemäße Mittel eignet sich besonders zur Anwendung in Werkstätten, Garagen, Tankstellen oder dergleichen, um in einfacher und schneller Weise festzustellen, ob die hydraulische Flüssigkeit, insbesondere Bremsflüssigkeit, aufgrund ihres Wassergehalts gegebenenfalls sofort erneuert werden muß oder noch gefahrlos weiter verwendet werden kann.

In analoger Weise zu dem oben beschriebenen Amaranth sind auch Azofarbstoffe wirksam, deren Azogruppe mit zwei Naphthylen- oder einer Naphthylen- und einer Phenylengruppe substituiert ist, wobei diese durch je eine oder zwei Sulfonsäuregruppen wasserlöslich gemacht sind und gegebenenfalls noch eine oder mehrere Hydroxyl- und/oder Aminogruppe(n) tragen können, wie aus der oben angegebenen allgemeinen Formel für die erfindungsgemäß anzuwendenden Indikatormittel hervorgeht.

Die nachfolgenden Beispiele sollen die Variante a) nach der Erfindung näher erläutern:

B e i s p i e l 1

Verwendung des Farbindikators in einer Flüssigkeit

Zunächst wird eine konzentrierte Farbindikatorlösung hergestellt durch Auflösen von 10 g Diphenylcarbazon und 10 g 1,10-Phenanthroliniumchlorid in 1 l Aceton. Von dieser konzentrierten Lösung werden 0,6 $cm^3$ mit 20 $cm^3$ Isooctan gemischt. Die erhaltene Mischung wird zu etwa der gleichen Menge, d. h. zu 20 $cm^3$, Bremsflüssigkeit gegeben, indem man die rot-violett gefärbte Indikatorflüssigkeit vorsichtig auf die Bremsflüssigkeit überschichtet. Bei Anwesenheit von Wasser tritt bei dieser an der Berührungsfläche zur Bremsflüssigkeit eine Entfärbung ein. Die Bremsflüssigkeit enthält dann mehr als 2 % Wasser. Sie muß in der Bremseinrichtung erneuert werden.

B e i s p i e l 2

Verwendung des Farbindikators auf einem Papierstreifen

Die gemäß Beispiel 1 hergestellte konzentrierte Farbindikatorlösung in Aceton wird zur Imprägnierung von Filterpapier verwendet, indem man das Indikatorblatt in die Indikatorlösung eintaucht, dann wieder herauszieht und trocknen läßt, worauf das imprägnierte, hellrosa gefärbte Indikatorpapier in ca. 0,5 - 1 cm breite Streifen geschnitten wird. Diese werden dann unmittelbar zum Eintauchen in die zu untersuchende Bremsflüssigkeit verwendet. Bei Abwesenheit von Wasser tritt bei Benetzung mit der Bremsflüssigkeit eine rote Verfärbung ein. Bei einem Wassergehalt von ca. 2 % oder mehr bleibt die hellrosa Färbung bestehen bzw. hellt sich nach kurzer Zeit der Indikatorstreifen wieder zur hellrosa Färbung auf oder wird sogar praktisch farblos.

Sinngemäß erfolgt auch die Variante b), indem man anstelle der oben angegebenen Indikator-Komponenten die erfindungsgemäß brauchbaren Azo-Farbstoffe in Dispersion als solche anwendet oder diese - wie oben ausgeführt - in einen Prüfstreifen einbringt und diesen dann mit der zu untersuchenden hydraulischen Flüssigkeit in Berührung bringt.

0078544

PATENTANWÄLTE
WUESTHOFF-v. PECHMANN-BEHRENS-GOETZ
EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

D-8000 MÜNCHEN 90
SCHWEIGERSTRASSE 2
TELEFON: (089) 66 20 51
TELEGRAMM: PROTECTPATENT
TELEX: 524 070

EP-56 697
Anm.: Lucas Industries public limited company

## Patentansprüche

1. Mittel zur Anzeige eines kritischen Wassergehalts in Hydraulikflüssigkeiten, insbesondere in Bremsflüssigkeiten, auf der Basis von Glykolen, Polyglykolen oder Polyglykolethern, dadurch gekennzeichnet, daß es

a) einen Indikator in Form einer Lösung von Diphenylcarbazon und 1,10-Phenanthrolin bzw. dessen Additionssalz enthält oder

b) Monoazo-, Disazo- und Trisazo-Farbstoffe der allgemeinen Formel

$$(MO_3S)_x\text{-Ar-N=N-Ar}_1(SO_3M)_y\text{-Z},$$

worin Ar, $Ar_1$ und $Ar_2$ eine Phenylen- oder Naphthylengruppe, gegebenenfalls substituiert mit Hydroxyl- und/oder Aminogruppe(n), Z ein Wasserstoffatom oder die Gruppe $\text{-N=N-Ar}_2(SO_3M)_z$ bedeutet, M Na, K oder $NH_4$ sein kann und x, y und z 0 bis 3 ist, die in Gegenwart von etwa 5 % $H_2O$, insbesondere 2 % $H_2O$ eine Farbänderung erfahren.

2. Mittel nach Anspruch 1 a), dadurch g e k e n n z e i c h n e t , daß die Indikatorlösung aus einem flüssigen Kohlenwasserstoff enthaltend 0,003 - 0,045 Gew.-% Diphenylcarbazon und 0,003 - 0,01 Gew.-% Phenanthrolin oder dessen Additionssalz besteht.

3. Mittel nach Anspruch 1 b), dadurch g e k e n n z e i c h n e t , daß der Farbstoff Amaranth ist.

4. Mittel nach Anspruch 1 bis 3, dadurch g e k e n n z e i c h n e t , daß sich die Indikatorlösung bzw. der Farbstoff in einem Prüfstreifchen, insbesondere aus saugendem Papier, befindet.

5. Verfahren zur Anzeige eines kritischen Wassergehalts in Hydraulikflüssigkeiten, insbesondere in Bremsflüssigkeiten, auf der Basis von Glykolen, Polyglykolen oder Polyglykolethern, dadurch g e k e n n z e i c h n e t , daß man die Flüssigkeit mit der Indikatorlösung aus Anspruch 1 a) überschichtet.

6. Verfahren zur Anzeige eines kritischen Wassergehalts in Hydraulikflüssigkeiten, insbesondere in Bremsflüssigkeiten, auf der Basis von Glykolen, Polyglykolen oder Polyglykolethern, dadurch g e k e n n z e i c h n e t , daß man die Flüssigkeit mit dem Farbstoff aus Anspruch 1 b) in Berührung bringt.

7. Verfahren nach Anspruch 5 oder 6, dadurch g e k e n n z e i c h n e t , daß man den Prüfstreifen nach Anspruch 4 in die Flüssigkeit eintaucht.

0078544

Europäisches Patentamt

# EUROPÄISCHER RECHERCHENBERICHT

Nummer der Anmeldung

EP 82 11 0130

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| Y | CHEMICAL ABSTRACTS, Band 84, Nr. 24, 14. Juni 1976, Seite 152, Nr. 167207u, Columbus, Ohio, USA & JP - A - 75 81393 (NIKKO PITTSUKUSU K.K.) 02.07.1975 * Zusammenfassung * | 1,4 | G 01 N 33/28<br>G 01 N 31/22 |
| Y | US-A-3 898 172 (R.B. REIF et al.) * Insgesamt * | 1,4 | |
| A | DE-A-1 961 811 (ESSO RESEARCH) | | |
| Y | CHEMICAL ABSTRACTS, Band 74, Nr. 10, 8. März 1971, Seite 107, Nr. 44093q, Columbus, Ohio, USA & SU - A - 74 532 (I.P. BUDAROV) 26.08.1970 * Zusammenfassung * | 4 | RECHERCHIERTE SACHGEBIETE (Int. Cl. ³)<br>G 01 N |
| Y | US-A-3 505 020 (D.A. CALDWELL) * Insgesamt * | 1 | |
| A | | 6 | |

Der vorliegende Recherchenbericht wurde für alle Patentansprüche erstellt.

| Recherchenort | Abschlußdatum der Recherche | Prüfer |
|---|---|---|
| DEN HAAG | 08-02-1983 | MEYLAERTS H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82